(19) Europäisches Patentamt
European Patent Office
Office européen des brevets

(11) Publication number: 0 255 980 A2

# (12) EUROPEAN PATENT APPLICATION

(21) Application number: 87201607.6

(22) Date of filing: 25.06.84

(51) Int. Cl.4: C 07 D 311/16

(30) Priority: 05.07.83 JP 122222/83

(43) Date of publication of application: 17.02.88 Bulletin 88/07

(84) Designated Contracting States: DE FR GB

(60) Publication number of the earlier application in accordance with Art. 76 EPC: 0 130 833

(71) Applicant: YAMANOUCHI PHARMACEUTICAL CO. LTD.
No. 3-11 Nihonbashi-Honcho, 2-chome Chuo-ku
Tokyo (JP)

(72) Inventor: Kijima, Tadao
No. 1086-2 Oaza Sanagaya
Shiroka-cho (JP)

Kageyama, Shunji
No. 36-6, Nakano 1-chome
Nakano-ku Tokyo (JP)

Okada, Minoru
No. 10-20, Higashioi 6-chome
Shinagawa-ku Okyo (JP)

Ohata, Isao
No. 47-17, Marugasaki-cho
Omiya-shi Saitama (JP)

(74) Representative: Geering, Keith Edwin et al
REDDIE & GROSE 16 Theobalds Road
London WC1X 8PL (GB)

A request for correction of claim 1 has been filed pursuant to Rule 88 EPC. A decision on the request will be taken during the proceedings before the Examining Division (Guidelines for Examination in the EPO, A-V, 2.2).

## (54) Coumarin intermediates for preparation of pharmaceuticals.

(57) Compounds of formula III:

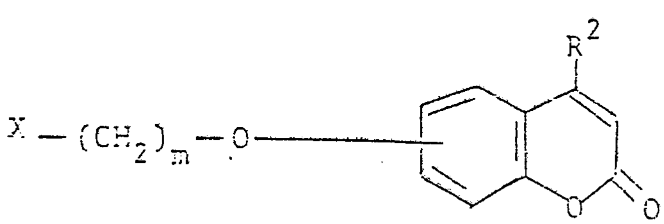

wherein X represents halogen atom; $R^2$ represents a hydrogen atom of an alkyl group; and m represents an integer of 1 to 2, can be used to prepare pharmaceutical compounds wherein X is an imidazolyl or pyridyloxy group optionally substituted by alkyl group(s).

EP 0 255 980 A2

**Description**

COUMARIN INTERMEDIATES FOR PREPARATION OF PHARMACEUTICALS

This invention relates to intermediates for producing coumarin compounts shown by the formula (I), and the salts thereof:

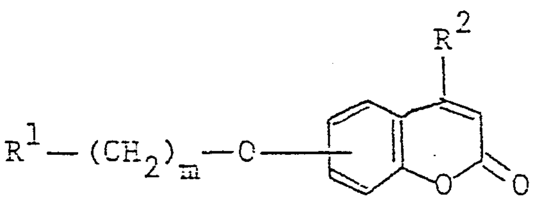

(I)

wherein $R^1$ represents an imidazolyl group or a pyridyloxy group which may be substituted by alkyl group(s); $R^2$ represesnts a hydrogen atom or alkyl group, and m represents an integer of 1 to 6. Any alkyl group will usually be a lower alkyl group.

The compounds according to the inventions are of formula (III):

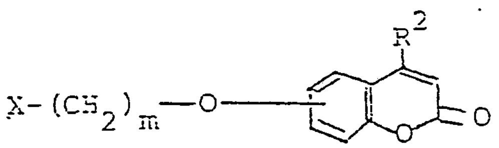

wherein X is halogen and $R^2$ is as defined above. $R^1$, $R^2$ and m have the above-defined significance throughout.

"Lower alkyl group" as used in this specification includes straight chain and branched chain alkyl groups having from 1 to 5 carbon atoms, such as methyl group, ethyl group, propyl group, isopropyl group, butyl group, isobutyl group, sec-butyl group, tert-butyl group, pentyl group (amyl group), isopentyl group, neopentyl group, tert-pentyl group, etc.

Some typical compounds of formula (I) are as follows:

7-[5-(1-imidazolyl)pentyloxy]coumarin
7-[5-(6-methyl-3-pyridyloxy)pentyloxy]coumarin
7-[5-(3-pyridyloxy)pentyloxy]coumarin
7-[3-(1-imidazolyl)propoxy]coumarin
7-[5-(6-ethyl-3-pyridyloxy)pentyloxy]coumarin
7-[4-(6-methyl-3-pyridyloxy)butoxy]coumarin
7-[4-(1-imidazolyl)butoxy]coumarin
7-[3-(6-methyl-3-pyridyloxy)propoxy]coumarin
7-[5-(1-imidazolyl)ethoxy]-4-methylcoumarin
7-[(1-imidazolyl)methoxy]coumarin

The compounds of the above formula (I) can form salts. Pharmacologically acceptable such salts include those with inorganic acid such as hydrochloric acid, sulfuric acid, hydrobromic acid, phosphoric acid, etc.; those with an organic acid such as formic acid, acetic acid, lactic acid, oxalic acid, succninic acid, fumaric acid, maleic acid, methansulfonic acid, ethansulfonic acid, benzenesulfonic acid, etc.; and also the quaternary ammonium salts obtained by reaction with an alkyl halide - e.g. methyl iodide.

The compounds of this formula (I) have platelet aggregation inhibiting activity, and are effective for the prophylaxis and the medical treatment of arteriosclerosis, cerebral embolism, cerebral infarction, transient paroxysmal ischemia, angina pectoris, peripheric thrombus and peripheric embolism.

The parmacological effects of compounds of formula (I) were examined in the following experiment.

Platelet aggregation inhibiting activity:

Platelet-rich plasma (PRP) and platelet-poor plasma (PPP) used for the experiment were obtained from Japan white rabbit's venous blood. Platelet aggregation rate and extent were determined in accordance with a method described in Born, G.V.R.; Nature; 194, 927(1962). The effects of compounds of this invention in inhibiting platelet aggregation induced by adding arachidonic acid (final concentration: 0.3 mM) were measured by Paytons aggregometer. The results showed that the compounds of this invention possess inhibiting activity, and that the effective concentration is $IC_{50}$: 10-100 μM.

The compounds of this formula (I) be formed into various pharmaceutical formulations such as powder, granule, tablet, capsule, injection, etc., using conventional carriers. The compounds can be administered orally (tablet, capsule, granule, powder, liquid, etc.) or parenterally (intravenous injection or intramuscular injection, suppository, etc.). The dosage of the compounds depends upon the condition, age, etc. of the patient, but for

oral administration the adult dosage is usually 1-100 (preferably 5-25 mg/kg) a day, which can for example be administered all at once or in 2 to 4 divided doses.

The compounds of formula I are prepared as follows:

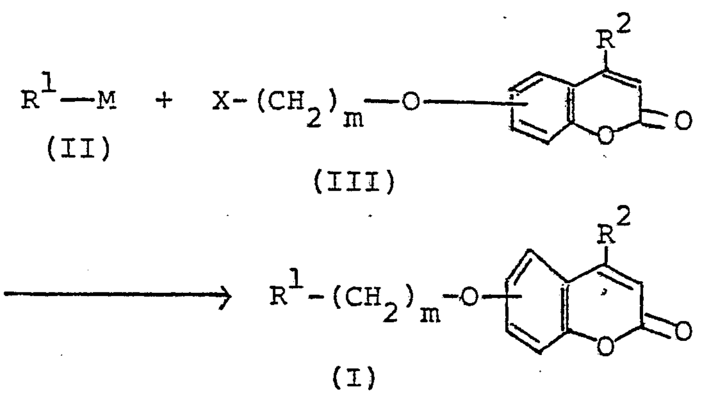

(II) (III) (I)

wherein M represents a hydrogen atom or alkali metal atom (e.g. Na or K), and X represents a halogen atom (e.g.Cℓ, Br or I) ; M and X have the above-defined significance throughout this specification.

Thus, a compound of formula (I) can be prepared by reacting an imidazole compound or hydroxypyridine compound (M=H) shown by formula (II) with a halogenoalkyloxycoumarin compound of formula (III) in the presence of a base or a basic material. A compound of formula (I) can be also prepared by reacting an alkali metal salt of said imidazole compound or of said hydroxypyridine compound (M=alkali metal) with a compound of formula (III).

It is preferred that the reaction (that is, N-alkylation or O-alkylation) be carried out at room temperature or under heating in an organic solvent such as alcohol (methanol, ethanol, etc.), dimethylsulfoxide, dimethylformamide, benzene, toluene, xylene, ether, tetrahydrofuran, etc.

Suitable base or basic material for use in the direction reaction between imidazole or hydroxypyridine (II, M=H) and the compound (III) includes potassium carbonate, sodium hydroxide, sodium amide, alkali metal alcoholate such as sodium ethoxide, sodium hydride, etc. , and organic lithium compound such as n-butyl lithium, etc.

The compounds of formula (I) prepared as above can be isolated and purified, as the free compounds or the salts thereof, by conventional chemical operations such as recrystallization, extraction, distillation, concentration, various chromatographies, and crystallization, etc.

The present invention is illustrated, without limitation, by the following Examples.

Example 1.

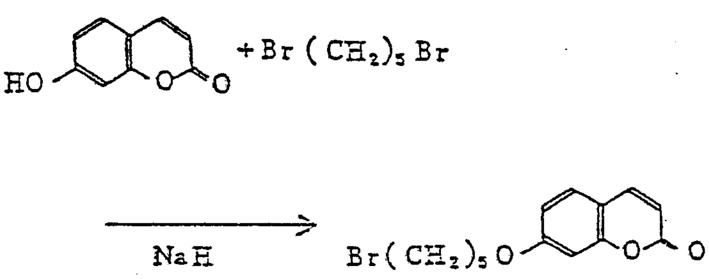


After 4.0 g of sodium hydride (60% suspension in a mineral oil) was washed with dry benzene, 100 ml of dry dimethylformamide was added thereto. While stirring at room temperature, 16.2 g of 7-hydroxycoumarin was added to the mixture. After vigorous bubbling was over, the resulting suspension was heated at 80°C for 30 minutes while stirring and then cooled to room temperature. A solution of 15 ml of 1,5-dibromopentane in 30 ml of dry N,N′-dimethylformamide was added thereto, and heating was conducted at 60°C for 4 hours while stirring. The solvent was removed from the reaction liquid by distillation under reduced pressure. The residue was dissolved in methylene chloride and the solution was successively washed with a 5 % aqueous sodium hydrogen carbonate solution, water and then a saturated aqueous sodium chloride solution followed by drying over anhydrous sodium sulfate. After drying the solvent was distilled off under reduced pressure. The oily residue was subjected to silica gel column chromatography and the product was eluted using a benzene:n-hexane mixture (1 : 5) as eluant. The solvent was removed from the elute by distillation under reduced pressure to obtain the compound 7-(5-bromopentyloxy)coumarin, as an oily substance.

NMR Spectrum ($CDCl_3$) (internal standard: TMS)

δ (ppm) 1.4—2.2 (m, 6H, C—$(CH_2)_3$—C)

3.46 (t, 2H, Br—$CH_2$—C, J=6.5Hz)

4.03 (t, 2H, 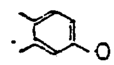 —$CH_2$—C, J=6.5Hz)

6.24 (d, 1H, 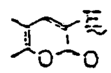 J=10.0Hz)

6.78 (d, 1H, 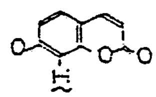 J=1.8Hz)

6.83 (dd, 1H, 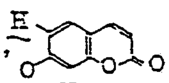 J=9.0Hz, 1.8Hz)

7.36 (d, 1H, 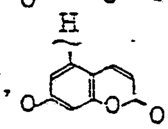 J=9.0Hz)

7.62 (d, 1H, 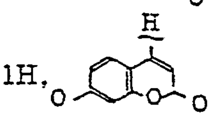 J=10.0Hz)

Example 1a

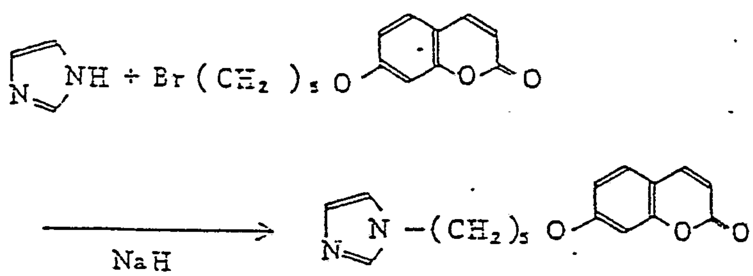


After 0.4 g of sodium hydride (60% suspension in a mineral oil) was washed with dry benzene, 30 ml of dry N,N′-dimethylformide was added thereto. While stirring at room temperature, 0.7 g of imidazole was added to the mixture. After vigorous bubbling was over, the resulting suspension was heated at 80° C for 30 minutes while stirring and then cooled to room temperature. A solution of 3.1 g of 7-(5-bromopentyloxy)coumarin in 10 ml of dry N,N-dimethylformamide was added thereto, and heating was conducted at 60°C for 4 hours while stirring. The solvent was removed from the reaction liquid by distillation under reduced pressure. The residue was dissolved in methylene chloride and the solution was successively washed with a 5% aqueous sodium hydrogen carbonate solution, water and then a saturated aqueous sodium chloride solution followed by drying over anhydrous sodium sulfate. After drying, the solvent was distilled off under reduced pressure. The residual oily substance was subjected to silica gel column chromatography and the product was eluted using chloroform:methanol (99:1) as an eluant. The solvent was removed from the elute by distillation under reduced pressure to obtain the compound 7-[5-(1-imidazolyl)pentyloxy]coumarin as an oily substance. The hydrochloride salt of this compound has a melting point of 134-135° C after recrystallization from ethanol.



BAD ORIGINAL

Elemental Analysis (as $C_{17}H_{19}N_2O_3Cl$)

| | C(%) | H(%) | N(%) | Cl(%) |
|---|---|---|---|---|
| Calculated: | 60.99 | 5.72 | 8.37 | 10.59 |
| Found: | 60.73 | 5.88 | 8.36 | 10.30 |

Example 2

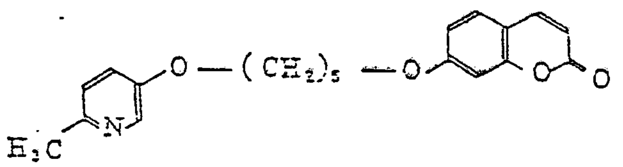

In accordance with the same procedure as in Example 1a, using 6-methyl-3-hydroxypyridine instead of imidazole, 7-[5-(6-methyl-3-pyridyloxy)pentyloxy]coumarin was obtained after recrystallizaiton from ethyl acetate.
Melting point: 101-102°C

Elemental Analysis (as $C_{20}H_{21}NO_4$)

| | C(%) | H(%) | N(%) |
|---|---|---|---|
| Calculated: | 70.78 | 6.24 | 4.13 |
| Found: | 70.51 | 6.35 | 4.09 |

Example 3

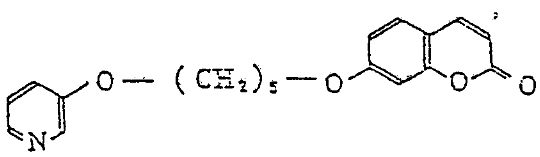

In accordance with the same procedure as in Example 1a, using 3-hydroxypyridine instead of imidazole, 7-[5-(3-pyridyloxy)pentyloxy]coumarin was obtained after recrystallization from ethyl acetate.
Melting point: 88-89°C

Elemental Analysis (as $C_{19}H_{19}NO_4$)

| | C(%) | H(%) | N(%) |
|---|---|---|---|
| Calculated: | 70.14 | 5.89 | 4.30 |
| Found: | 70.03 | 5.83 | 4.30 |



BAD ORIGINAL

## Claims

1. A coumarin compound of the following general formula,

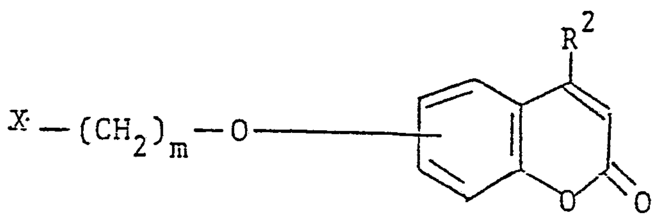

wherein X represents halogen atom; $R^2$ represents a hydrogen atom or an alkyl group; and m represents an integer of 1 to 2.

2. A compound according to Claim 1 wherein m is 5, X is Br and $R^2$ is a hydrogen atom.